# EUROPÄISCHE PATENTSCHRIFT

(11) **EP 2 167 393 B1**
(45) Veröffentlichungstag und Bekanntmachung des Hinweises auf die Patenterteilung: **22.06.2011**
(21) Anmeldenummer: 08759323.2
(22) Anmeldetag: 24.06.2008
(51) Int. Cl.: B65D 23/00, A61M 5/14

(54) **VORFORMLING UND VERFAHREN ZUR HERSTELLUNG EINES BEHÄLTNISSES ZUR AUFNAHME VON FLÜSSIGKEITEN FÜR MEDIZINISCHE ANWENDUNGEN**
PREFORM AND METHOD FOR PRODUCING A CONTAINER FOR HOLDING FLUIDS USED IN MEDICAL APPLICATIONS
ÉBAUCHE ET PROCÉDÉ DE FABRICATION D'UN CONTENANT DESTINÉ À RECEVOIR DES LIQUIDES POUR DES APPLICATIONS MÉDICALES

(30) Priorität: 27.06.2007 DE 102007029810
(43) Veröffentlichungstag der Anmeldung: 31.03.2010
(73) Patentinhaber: Fresenius Kabi Deutschland GmbH, 61352 Bad Homburg v.d.H. (DE)
(72) Erfinder: BRANDENBURGER, Torsten, 61203 Reichelsheim (DE); GREIER, Gerhard, 61381 Friedrichsdorf (DE); RAHIMY, Ismael, 61169 Friedberg (DE)
(74) Vertreter: Luderschmidt, Schüler & Partner
(86) Internationale Anmeldenummer: PCT/EP2008/005064
(87) Internationale Veröffentlichungsnummer: WO 2009/000492

(56) Entgegenhaltungen:
- EP-A- 0 483 671
- JP-A- 62 273 822
- JP-A- 62 273 823
- JP-A- 63 011 324
- US-A- 4 395 378
- US-A- 4 932 566
- US-A1- 2006 145 041

## Beschreibung

Die Erfindung bezieht sich auf einen Vorformling zur Herstellung eines Behältnisses zur Aufnahme von Flüssigkeiten für medizinische Anwendungen, insbesondere zur Aufnahme von Infusionslösungen oder enteralen Nährlösungen. Darüber hinaus betrifft die Erfindung ein Verfahren zur Herstellung eines Behältnisses zur Aufnahme von Flüssigkeiten für medizinische Anwendungen, insbesondere von Infusionslösungen oder enteralen Nährlösungen. Des Weiteren betrifft die Erfindung ein Behältnis zur Aufnahme von Flüssigkeiten für medizinische Anwendungen.

Es ist eine Vielzahl von Behältnissen unterschiedlicher Ausbildung zur Aufnahme von Flüssigkeiten bekannt. Behältnisse zur Aufnahme von Flüssigkeiten für medizinische Behandlungen müssen besonderen Anforderungen, insbesondere an die Sterilität genügen.

Es sind Behältnisse für medizinische Flüssigkeiten, beispielsweise Flaschen aus extrudiertem PE oder PP bekannt, die in einem Arbeitsgang steril und pyrogenfrei durch biaxiales Strecken und Blasformen eines Vorformlings, der auch als Preform bezeichnet wird, in die gewünschte Form gebracht werden, nach der Abkühlung mit einem sterilen Füllgut aseptisch befüllt und anschließend hermetisch verschlossen werden. Diese nach dem Blasen-Füllen-Verschließen-Verfahren (Blow-Fill-Seal-Verfahren) hergestellten Behältnisse, insbesondere Flaschen, werden auch als PFS-Behälter bezeichnet.

Die bekannten Behältnisse für Infusionslösungen oder enterale Nährlösungen werden im Allgemeinen an einem Ständer aufgehängt. Dafür verfügen die Behältnisse über einen Aufhänger. Es sind zahlreiche Behälter bekannt, die über einen Aufhänger verfügen, der einstückiger Bestandteil des Behälters ist. Die EP 070 641 A1 und die US 3 901 399 beispielsweise beschreiben Flaschen, an deren Bodenteil Aufhänger angeformt sind. JP 62273823A öffenbart ein Vorformling mit einem ringförmigen Aufhänger. Die Aufhänger sind als Ösen ausgebildet, um die Flasche an einem Haken aufhängen zu können.

Die EP 0 483 671 B1 beschreibt ein Verfahren zur Herstellung eines Infusionsbehältnisses, das über einen ringförmigen Aufhänger am Boden der Flasche verfügt. Die Flasche wird durch biaxiales Strecken und Aufblasen aus einem Vorformling (Preform) hergestellt. Der Vorformling selbst wird zusammen mit dem ringförmigen Aufhänger im Spritzgießverfahren gefertigt. Das bekannte Herstellungsverfahren zeichnet sich dadurch aus, dass ein weiterer Arbeitsschritt zur Befestigung eines Aufhängers nach dem Blasformen nicht erforderlich ist.

Der Erfindung liegt die Aufgabe zu Grunde, einen Vorformling bereitzustellen, mit dem ein Behältnis zur Aufnahme von Flüssigkeiten für medizinische Anwendungen, insbesondere zur Aufnahme von Infusionslösungen oder enteralen Nährlösungen auf einfache Weise mit einem Aufhänger hergestellt werden kann. Darüber hinaus ist eine Aufgabe der Erfindung, ein einfach durchzuführendes Verfahren zur Herstellung eines Behältnisses zur Aufnahme von medizinischen Flüssigkeiten anzugeben, dass über einen Aufhänger verfügt. Eine weitere Aufgabe der Erfindung ist, ein einfach herzustellendes Behältnis zur Aufnahme von medizinischen Flüssigkeiten bereitzustellen.

Die Lösung dieser Aufgabe erfolgt erfindungsgemäß mit den Merkmalen der Patentansprüche 1, 14 und 18. Vorteilhafte Ausführungsformen der Erfindung sind Gegenstand der Unteransprüche.

Der erfindungsgemäße Vorformling zur Herstellung eines Behältnisses für medizinische Flüssigkeiten, der einen Halsteil mit einer Öffnung, einen Wandteil und einen Bodenteil aufweist, zeichnet sich dadurch aus, dass an der Unterseite des Bodenteils das eine Ende eines ersten und zweiten Teilstücks angeformt ist, die nach der Herstellung des Vorformlings zu einem ringförmigen Aufhänger zum Aufhängen des Behältnisses miteinander verbunden werden können.

Zunächst wird der Vorformling hergestellt, wobei die freien Enden der beiden Teilstücke noch nicht miteinander verbunden sind. Anschließend wird das Behältnis durch Strecken und Blasformen aus dem Vorformlings geformt. Die hierzu erforderlichen Verfahrensschritte sind dem Fachmann bekannt.

Der Vorformling selbst wird vorzugsweise im Spritzgießverfahren hergestellt. Die hierzu erforderlichen Verfahrensschritte sind dem Fachmann ebenfalls bekannt. Entscheidend ist, dass beim Spritzgießen des Vorformlings die beiden Teilstücke des ringförmigen Aufhängers noch nicht miteinander verbunden sind. Dadurch wird das Spritzgießverfahren zur Herstellung des Vorformlings vereinfacht. Es ergibt sich eine relativ hohe Füllgeschwindigkeit des Formwerkzeugs im Bereich des Aufhängers aufgrund eines geringeren Anspritzdurchmessers durch zwei Anspritzpunkte, so dass sich die Zykluszeiten verkürzen. Weiterhin ergibt sich eine genaue Wandstärkenverteilung und schnelle Kühlung und damit ein besseres Lösen vom Formwerkzeug. Ferner wird das gesamte Volumen der Flasche ausgenutzt.

Die Verbindung der beiden Teilstücke des Aufhängers kann nach dem Spritzgussprozess, beispielsweise während des Streckblasens oder auch erst später beim Kunden erfolgen.

Bei einer bevorzugten Ausführungsform des Vorformlings sind das erste und zweite Teilstück als flexible Streifen ausgebildet. Die zur Verformung der Teilstücke zu dem ringförmigen Aufhänger erforderliche Flexibilität kann durch die Auswahl eines Materials mit ausreichender Flexibilität und/oder durch eine geeignete Dimensionierung des Querschnitts der Teilstücke erreicht werden.

Eine besonders bevorzugte Ausführungsform sieht vor, dass die flexiblen Teilstücke als flache Streifen mit einem rechteckförmigen Querschnitt ausgebildet sind. Dadurch ist auch bei einem weniger flexiblen Material eine ausreichende Flexibilität gegeben. Auf jeden Fall sollte das Material so flexibel sein, dass es beim Biegen nicht bricht. Anstelle eines rechteckförmigen Querschnitts ist aber auch ein kreisförmiger Querschnitt möglich.

Eine weitere besonders bevorzugte Ausführungsform sieht vor, dass die an der Unterseite des Bodenteils angeformten Enden der Teilstücke ein gemeinsames Basisstück bilden. Das Basisstück ist vorzugsweise als flacher Körper ausgebildet, der an die Unterseite des Bodenteils angeformt ist. Von dem flachen Körper erstrecken sich die übrigen Abschnitte der flexiblen Teilstücke nach außen. Dabei haben die beiden Teilstücke insbesondere in diesen Abschnitten eine ausreichende Flexibilität. Das an den Bodenteil angeformte Basisstück ist hingegen vorzugsweise im Wesentlichen starr.

Die Erfindung sieht zwei alternative Ausführungsformen vor, die sich dadurch unterscheiden, dass bei der ersten Ausführungsform die freien Enden der beiden Teilstücke als miteinander zu verschweißende Endstücke ausgebildet sind, während die andere Ausführungsform Teilstücke aufweist, deren freie Enden als formschlüssig miteinander zu verbindende Endstücke ausgebildet sind.

Bei der ersten Ausführungsform können die Stirnflächen der miteinander zu verschweißenden Endstücke entweder einander gegenüberliegend angeordnet sein, wobei die Teilstücke direkt an den Stirnflächen miteinander verschweißt werden, oder die zu verschweißenden Enden weisen zusätzliche Ansatzstücke mit einander zugewandten Anlageflächen auf, die miteinander verschweißt werden, so dass eine besonders feste Schweißverbindung erzielt wird. Eine weitere Ausführungsform sieht vor, dass nicht die freien Enden der beiden Teilstücke oder deren Ansatzstücke miteinander verschweißt werden, sondern das erste und zweite Teilstück einen ringförmiger Körper bilden, wobei sich die Teilstücke über einen Teil des Umfangs einander überlappen. Dadurch ist es möglich, die einander zugewandten Anlageflächen der sich überlappenden Teilstücke miteinander zu verschweißen, so dass wieder eine besonders feste Verbindung erzielt wird.

Bei der alternativen Ausführungsform, bei der die freien Enden der Teilstücke als formschlüssig miteinander zu verbindende Endstücke ausgebildet sind, können sich die Teilstücke parallel zueinander erstrecken oder ringförmig geformt sein. Grundsätzlich können aber auch bei der Ausführungsform mit den zu verschweißenden Enden die Teilstücke vor dem Verbinden geradlinig oder gebogen sein.

Eine besonders bevorzugte Ausführungsform sieht vor, dass die freien Enden der beiden Teilstücke als Schnapp- oder Rastverbindung ausgebildet sind, so dass die Verbindung der Teilstücke auch erst beim Kunden erfolgen kann.

Im Folgenden werden verschiedene Ausführungsbeispiele der Erfindung unter Bezugnahme auf die Zeichnungen näher erläutert.

Es zeigen:
- Fig. 1: ein erstes Ausführungsbeispiel des erfindungsgemäßen Vorformlings in geschnittener Darstellung, bei dem die freien Enden der Teilstücke miteinander verschweißt werden.
- Fig.2: einen Schnitt durch den Vorformling von Fig. 1,
- Fig. 3: eine perspektivische vergrößerte Darstellung des Aufhängers des Vorformlings von Fig. 1,
- Fig. 4: ein zweites Ausführungsbeispiel des Aufhängers des erfindungsgemäßen Vorformlings in vergrößerter perspektivischer Darstellung,
- Fig. 5: ein drittes Ausführungsbeispiel des Aufhängers in perspektivischer Darstellung,
- Fig. 6: ein viertes Ausführungsbeispiel des Aufhängers,
- Fig. 7: eine alternative Ausführungsform des Aufhängers, bei der die freien Enden der Teilstücke formschlüssig miteinander verbunden werden,
- Fig. 8: ein weiteres Ausführungsbeispiel des Aufhängers der alternativen Ausführungsform,
- Fig. 9: ein weiteres Ausführungsbeispiel der alternativen Ausführungsform des Aufhängers,

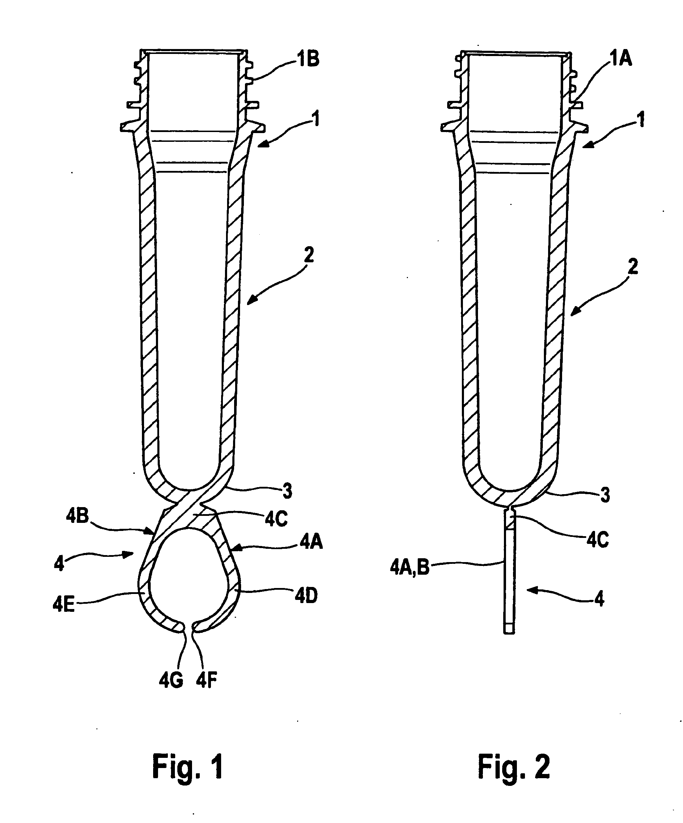

Die Figuren 1 und 2 zeigen in geschnittener Darstellung ein erstes Ausführungsbeispiel eines erfindungsgemäßen Vorformlings zur Herstellung eines Behältnisses zur Aufnahme einer medizinischen Flüssigkeit, insbesondere einer Infusionslösung oder enteralen Nährlösung. Der Vorformling wird im Spritzgießverfahren, insbesondere im Spritzgießverfahren mit zwei bzw. drei Komponenten aus Kunststoff, insbesondere Polypropylen PP, Polyethylen PE, PET oder einer Kombination aus PP, PE, PET hergestellt, das eine ausreichende Stabilität hat. Der Vorformling weist einen Halsteil 1 mit einer Öffnung 1A auf, der mit einem Außengewinde 1B versehen ist. An den Halsteil 1 schließt sich ein Wandteil 2 an, das in einen Bodenteil 3 übergeht.

An der Unterseite des Bodenteils 3 des Vorformlings ist ein Kunststoffteil 4 angeformt, der später den Aufhänger bildet. Der Kunststoffteil 4 ist einstückiger Bestandteil des Vorformlings. Er besteht aus zwei Teilstücken 4A, 4B, die an die Unterseite des Bodenteils 3 angeformt sind.

Die an die Unterseite des Bodenteils angeformten Enden des ersten und zweiten Teilstücks bilden ein gemeinsames Basisstück 4C, das als flacher Körper ausgebildet ist. Die von dem flachen Basiskörper 4C ausgehenden Abschnitte 4D bzw. 4E der beiden Teilstücke 4A, 4B sind derart ringförmig gebogen, dass sich die Stirnflächen 4F, 4G der Teilstücke 4A, 4B einander dicht gegenüber liegen.

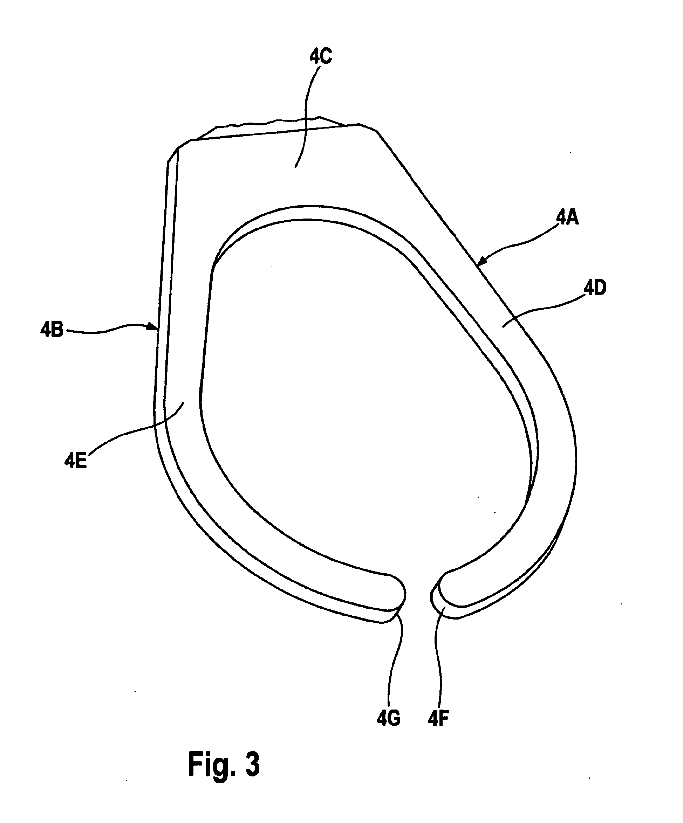

Fig. 3 zeigt den Aufhänger vor dem Verschweißen der freien Enden der Teilstücke in vergrößerter perspektivischer Darstellung. Die von dem flachen Basisstück 4C ausgehenden Abschnitte der beiden Teilstücke 4A, 4B sind flache flexible Streifen mit einem rechteckförmigen Querschnitt. Sie sind ausreichend flexibel, so dass die Teilstücke leicht zusammengebogen und die freien Enden durch Verschweißen der Stirnflächen 4F, 4G miteinander verbunden werden können. Nach dem Verschweißen der Enden bildet der Aufhänger eine im Wesentlichen runde oder ovale Öse.

Das Behältnis wird aus dem Vorformling durch biaxiales Strecken und Aufblasen nach den bekannten Verfahren geformt. Dabei bleibt die Form des Aufhängers unverändert. Die beiden Teilstücke des Aufhängers werden erst nach dem Spritzgießen des Vorformlings miteinander verschweißt. Dadurch wird das Spritzgießen vereinfacht. Der Schweißvorgang wird vorzugsweise während des Streckens und Aufblasens durchgeführt. Grundsätzlich ist es aber auch möglich, die beiden Teilstücke vor dem Strecken und Aufblasen, aber nach dem Spritzgießen miteinander zu verschweißen.

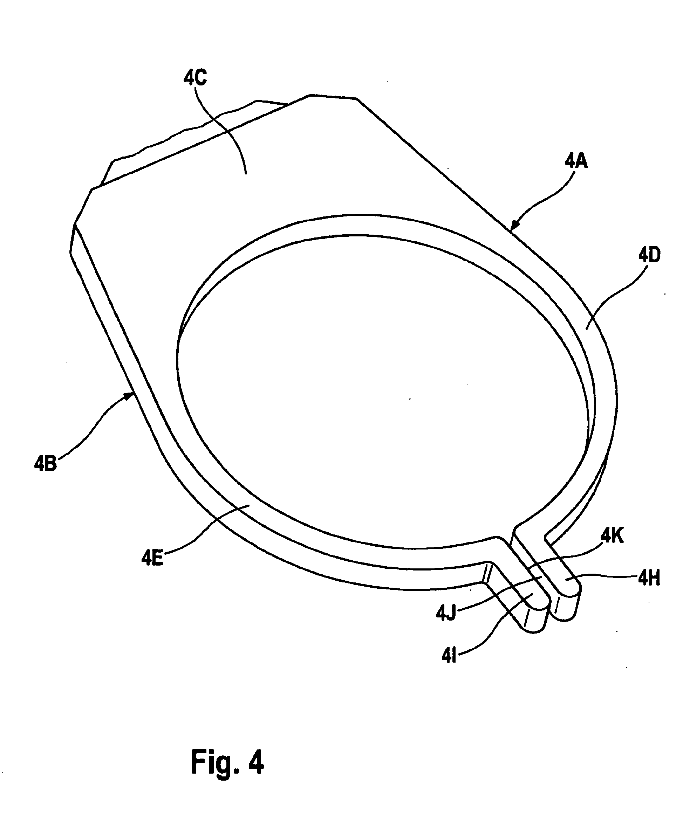

Fig. 4 zeigt in vergrößerter perspektivischer Darstellung ein zweites Ausführungsbeispiel des erfindungsgemäßen Aufhängers. Da sich bei dieser Ausführungsform Halsteil 1, Wandteil 2 und Bodenteil 3 des Vorformlings nicht von dem unter Bezugnahme auf die Fig. 1 bis 3 beschriebenen Vorformlings unterscheiden, kann auf die vollständige Darstellung des Vorformlings in Fig. 4 verzichtet werden. Die einander entsprechenden Teile sind mit den gleichen Bezugszeichen bezeichnet.

Das zweite Ausführungsbeispiel unterscheidet sich von dem ersten Ausführungsbeispiel dadurch, dass an den freien Enden der Teilstücke 4A, 4B nach außen vorstehende parallel zueinander verlaufende Ansatzstücke 4H, 4I angeformt sind. Die einander dicht gegenüberliegenden Anlageflächen 4J, 4K der beiden Ansatzstücke 4H, 4I werden miteinander verschweißt. Dadurch wird eine einfach herzustellende und besonders feste Schweißverbindung erzielt.

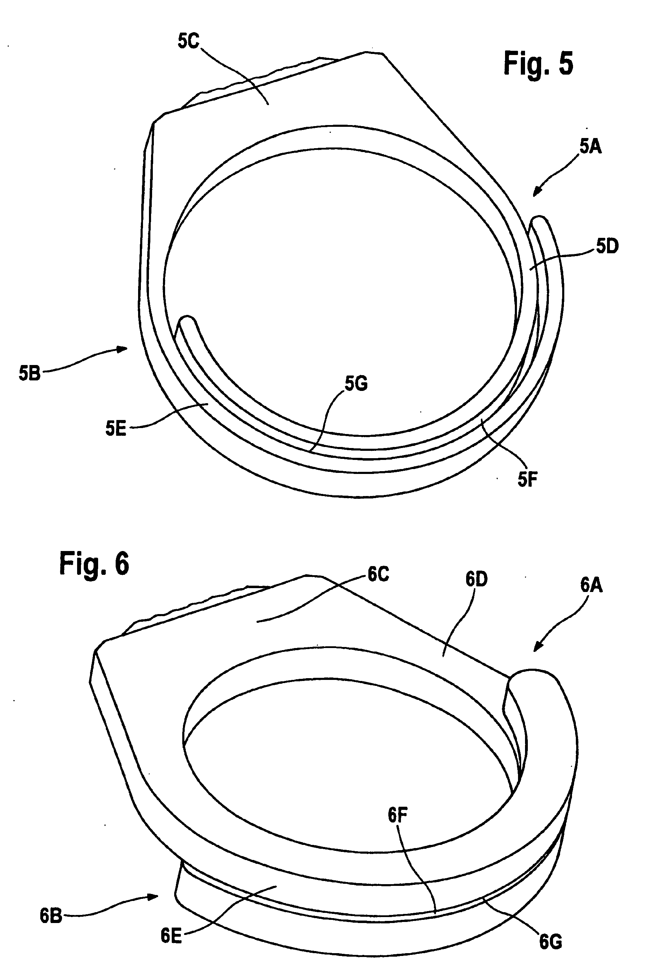

Fig. 5 zeigt ein drittes Ausführungsbeispiel des Aufhängers vor dem Verschweißen der beiden Teilstücke 5A, 5B, die in dem gemeinsamen flachen Basisstück 5C enden, das an die Unterseite des Bodenteils 3 des Vorformlings angeformt ist. Dieses Ausführungsbeispiel unterscheidet sich von dem ersten und zweiten Ausführungsbeispiel dadurch, dass die von dem Basisstück 5C ausgehenden Abschnitte 5D, 5E der beiden Teilstücke 5A, 5B derart ringförmig gebogen sind, dass sich die Abschnitte über einen bestimmten Umfangswinkel, der beispielsweise 180 Grad betragen kann, einander überlappen. Die beiden Teilstücke 5A, 5B werden im Überlappungsbereich an den beiden dicht einander gegenüberliegenden Anlageflächen 5F, 5G miteinander verschweißt.

Dadurch wird eine besonders feste Schweißverbindung erzielt. Darüber hinaus erhält der Aufhänger nach dem Verschweißen eine besonders große Stabilität, obwohl die Teilstücke für die Verformung flexibel sind.

Ein weiteres Ausführungsbeispiel zeigt Fig. 6, das sich von dem Ausführungsbeispiel von Fig. 5 dadurch unterscheidet, dass die beiden von dem flachen Teilstück 6C ausgehenden Abschnitte 6D, 6E der beiden Teilstücke 6A, 6B im Überlappungsbereich in der Bildebene nicht nebeneinander (Fig. 5), sondern übereinander liegen. Dabei haben die Teilstücke 6A, 6B ebenfalls einen rechteckförmigen Querschnitt, wobei die Teilstücke aber nicht mit der Schmalseite in der Bildebene nach oben bzw. unten liegend (Fig. 5), sondern mit den Breitseiten nach oben bzw. unten liegend gebogen sind. Die Verschweißung der beiden Teilstücke erfolgt im Überlappungsbereich zwischen der breiteren Oberseite 6F des unteren und der breiteren Unterseite 6G des oberen Teilstücks 6A, 6B.

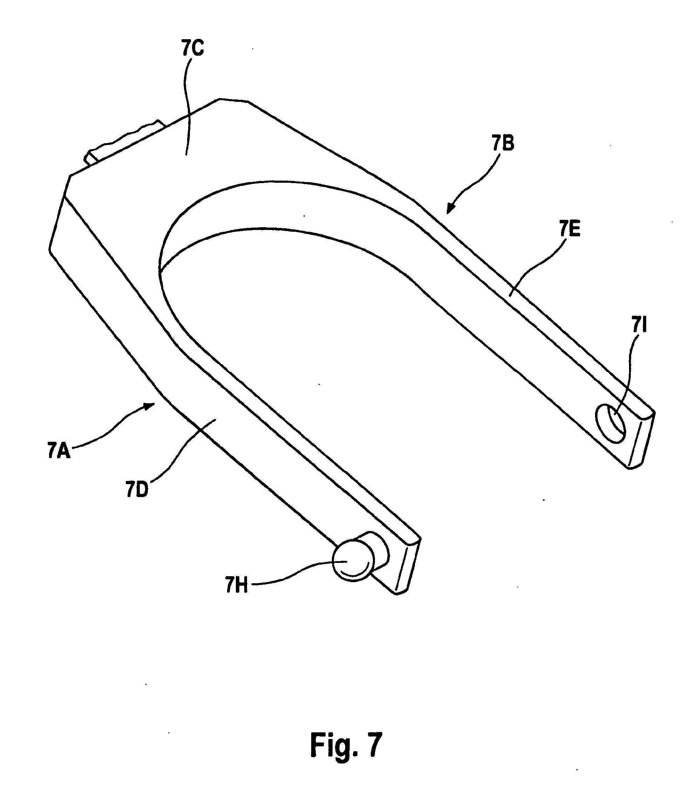

Unter Bezugnahme auf die Fig. 7 bis 9 werden nachfolgend weitere Ausführungsbeispiele alternativer Ausführungsformen beschreiben, die sich von den unter Bezugnahme auf die Fig. 1 bis 6 beschriebenen Ausführungsformen dadurch unterscheiden, dass die freien Enden der Teilstücke formschlüssig miteinander verbunden werden.

Fig. 7 zeigt ein Ausführungsbeispiel, bei dem die beiden Teilstücke 7A, 7B, die in dem gemeinsamen Basisstück 7C enden, die Form eines "U" bilden. Dabei verlaufen die von dem Basisstück 7C ausgehenden Abschnitte 7D, 7E der Teilstücke 7A, 7B parallel zueinander. Während an den freien Enden des einen Teilstücks 7A ein Schnapp- oder Rastelement 7H mit einem kugelförmigen Endstück angeformt ist, weist das freie Ende des anderen Teilstücks 7B ein Loch 7I auf, in das das Schnappelement 7H mit dem kugelförmigen Endstück einschnappend eingedrückt werden, so dass die Teilstücke des Aufhängers unverlierbar miteinander verbunden sind. Die Verbindung erfolgt nach dem Spritzgießen des Vorformlings vorzugsweise nach dem Formen des Behältnisses durch Strecken und Blasformen. Nach dem Verbinden der beiden Enden bildet der Aufhänger eine im Wesentlichen runde oder ovale Öse.

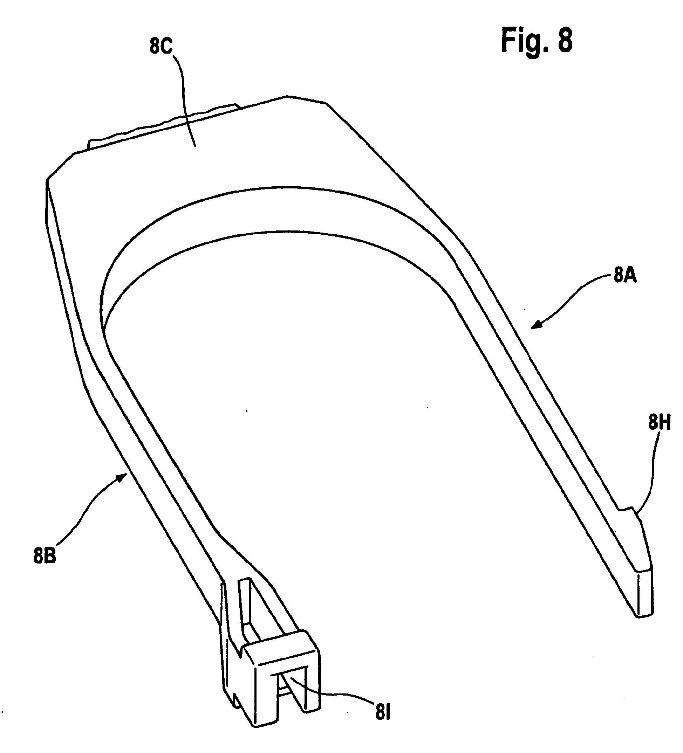

Fig. 8 zeigt ein weiteres Ausführungsbeispiel der alternativen Ausführungsformen. Die beiden Teilstücke 8A, 8B mit dem gemeinsamen Basisstück 8C erstrecken sich wieder parallel zueinander und bilden ein "U". Bei diesem Ausführungsbeispiel werden die freien Enden der Teilstücke 8A, 8B mittels einer Rast- oder Schnappverbindung miteinander verbunden. Das freie Ende des einen Teilstücks 8A weist einen hakenförmigen Körper 8H auf, der zum Ende hin flach ausläuft. Das freie Ende des anderen Teilstücks 8B verbreitert sich unter Ausbildung einer Ausnehmung 8I, die hinterschnitten ist. Zum Verbinden der beiden Teilstücke wird der hakenförmige Körper 8H in die hinterschnittene Ausnehmung 8I eingeschoben, wodurch beide Teile einrastend oder einschnappend miteinander verbunden sind.

Um die vor dem Verbinden parallel verlaufenden Teilstücke 9A, 9B leicht biegen zu können, sind die Teilstücke als flache flexible Streifen geformt, deren Schmalseiten in der Bildebene oben bzw. unten liegen.

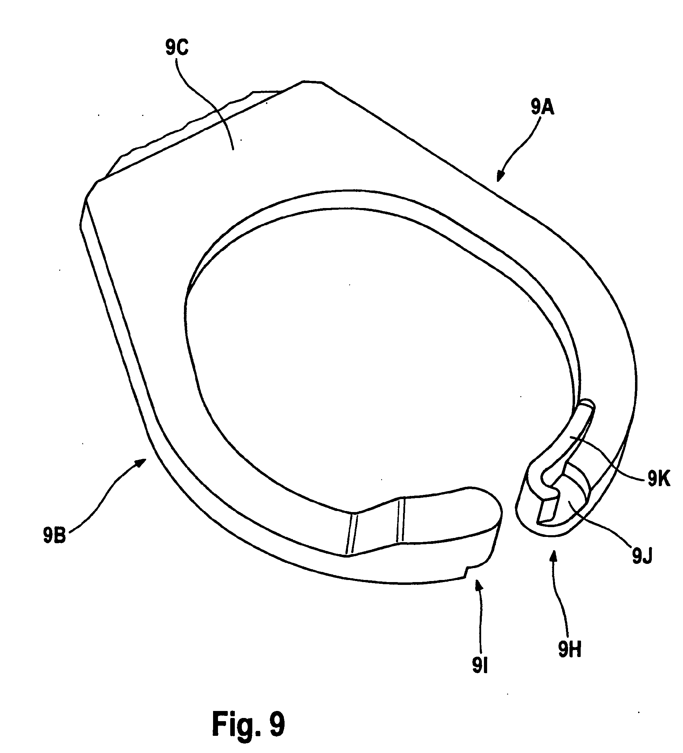

Fig. 9 zeigt ein weiters Ausführungsbeispiel der alternativen Ausführungsform, bei der die beiden Teilstücke 9A, 9B mit dem flachen Basisstück 9C bereits ringförmig geformt sind, so dass die Teilstücke nur leicht gebogen zu werden brauchen, um die freien Enden miteinander zu verbinden. Bei diesem Ausführungsbeispiel liegen die Breitseiten der Teilstücke 9A, 9B in der Bildebene oben bzw. unten, während die Schmalseiten nach außen oder innen weisen. Dadurch wird eine höhere Biegesteifigkeit als bei dem Ausführungsbeispiel von Fig. 8 erzielt. Die freien Enden der beiden Teilstück 9A, 9B sind als klauenförmige Körper 9H, 9I ausgebildet, die beim Zusammensetzen der Teilstücke formschlüssig ineinander greifen. Die klauenförmigen Körper 9H, 9I der Teilstücke 10A, 10B weisen einen flachen Steg 9J auf, an den ein dazu senkrecht stehender Steg 9K angeformt ist, der am Ende nach innen geschwungen ist.

## Patentansprüche

1. Vorformling zur Herstellung eines Behältnisses zur Aufnahme von Flüssigkeiten für medizinische Anwendungen, insbesondere zur Aufnahme von Infusionslösungen oder enteralen Nährlösungen, wobei der Vorformling einen Halsteil (1) mit einer Öffnung (1A), einen Wandteil (2) und einen Bodenteil (3) aufweist,
**dadurch gekennzeichnet, dass** an der Unterseite des Bodenteils (3) das eine Ende eines ersten Teilstücks (4A) und das eine Ende eines zweiten Teilstücks (4B) eines ringförmigen Aufhängers zum Aufhängen des Behältnisses angeformt sind, wobei die freien Enden des ersten und zweiten Teilstücks unter Verformung der Teilstücke zu dem ringförmigen Aufhänger miteinander verbindbar sind.

2. Vorformling nach Anspruch 1, **dadurch gekennzeichnet, dass** das erste und zweite Teilstück (4a, 4B) als flexible Streifen ausgebildet sind.

3. Vorformling nach Anspruch 2, **dadurch gekennzeichnet, dass** das die flexiblen Streifen (4a, 4B) als flache Streifen mit einem rechteckfömigen Querschnitt ausgebildet sind.

4. Vorformling nach einem der Ansprüche 1 bis 3, **dadurch gekennzeichnet, dass** die an die Unterseite des Bodenteils (3) angeformten Enden des ersten und zweiten Teilstücks (4a, 4B) ein gemeinsames Basisstück (4C) bilden, das an die Unterseite des Bodenteils angeformt ist.

5. Vorformling nach Anspruch 4, **dadurch gekennzeichnet, dass** das Basisstück (4C) als ein flacher Körper ausgebildet ist, der an die Unterseite des Bodenteils (3) angeformt ist.

6. Vorformling nach einem der Ansprüche 1 bis 5, **dadurch gekennzeichnet, dass** die freien Enden des ersten und zweiten Teilstücks (4a, 4B) als miteinander zu verschweißende Enden ausgebildet sind, deren Stirnflächen (4F, 4G) einander gegenüberliegend angeordnet sind.

7. Vorformling nach Anspruch 6, **dadurch gekennzeichnet, dass** die miteinander zu verschweißende Enden des ersten und zweiten Teilstücks (4A, 4B) jeweils ein Ansatzstück (4H, 4I) aufweisen, wobei die Ansatzstücke der Endstücke mit einander zugewandten Anlageflächen (4J, 4K) angeordnet sind.

8. Vorformling nach einem der Ansprüche 1 bis 5, **dadurch gekennzeichnet, dass** das erste und zweite Teilstück (5A, 5B; 6A, 6B) als ein ringförmiger Körper ausgebildet sind, wobei sich die Teilstücke über einen Teil des Umfangs einander überlappen, so dass die einander zugewandten Flächen der Teilstücke miteinander zu verschweißende Anlageflächen (5F, 5G; 6F, 6G) bilden.

9. Vorformling nach einem der Ansprüche 1 bis 5, **dadurch gekennzeichnet, dass** die freien Enden des ersten und zweiten Teilstücks (7A, 7B; 8A, 8B; 9A, 9B) als formschlüssig miteinander zu verbindende Endstücke ausgebildet sind.

10. Vorformling nach Anspruch 9, **dadurch gekennzeichnet, dass** die freien Enden des ersten und zweiten Teilstücks (7A, 7B; 8A, 8B) als formschlüssig miteinander zu verbindende Endstücke ausgebildet sind, wobei sich die Teilstücke parallel zueinander erstrecken.

11. Vorformling nach Anspruch 9, **dadurch gekennzeichnet, dass** die freien Enden des ersten und zweiten Teilstücks (9A, 9B) als formschlüssig miteinander zu verbindende Endstücke ausgebildet sind, wobei die Teilstücke ringförmig geformt sind.

12. Vorformling nach Anspruch 10 oder 11, **dadurch gekennzeichnet, dass** das Endstück des ersten Teilstücks (7A; 8A; 9A) und das Endstück des zweiten Teilstücks (7B; 8B; 9B) als eine Schnapp- oder Rastverbindung ausgebildet sind.

13. Vorformling nach Anspruch 12, **dadurch gekennzeichnet, dass** das Endstück des ersten Teilstücks (8A) einen hakenförmigen oder klauenförmigen Körper (8H) und das Endstück (8B) des zweiten Teilstücks (8A) eine hinterschnittene Ausnehmung (8I) aufweisen, wobei der haken- oder klauenförmige Körper des ersten Teilstücks in der hinterschnittene Ausnehmung des zweiten Teilstücks zur Verbindung der Teilstücke zu dem ringförmigen Aufhänger einsetzbar ist.

14. Herstellung eines Behältnisses zur Aufnahme von medizinischen Flüssigkeiten, insbesondere zur Aufnahme von Infusionslösungen oder Lösungen für die enterale Ernährung, mit folgenden Verfahrensschritten:
Bereitstellen eines Vorformlings nach einem der Ansprüche 1 bis 13,
Strecken und Blasformen des Vorformlings zu einem Behältnis,
Verbinden des ersten und zweiten Teilstücks zu einem ringförmigen Aufhänger.

15. Verfahren nach Anspruch 14, **dadurch gekennzeichnet, dass** die freien Enden des ersten und zweiten Teilstücks miteinander verschweißt werden.

16. Verfahren nach Anspruch 14, **dadurch gekennzeichnet, dass** die freien Enden des ersten und zweiten Teilstücks formschlüssig miteinander verbunden werden.

17. Verfahren nach einem der Ansprüche 14 bis 16, **dadurch gekennzeichnet, dass** der Vorformling im Spritzgießverfahren hergestellt wird.

18. Behältnis zur Aufnahme von medizinischen Flüssigkeiten, insbesondere zur Aufnahme von Infusionslösungen oder Lösungen für die enterale Ernährung, **dadurch gekennzeichnet, dass** das Behältnis nach dem Verfahren nach einem der Ansprüche 14 bis 17 hergestellt ist.

## Claims

1. A parison for the production of a receptacle used to hold fluids for medical applications, in particular to hold infusion solutions or enteral nutrient solutions, wherein the parison comprises a neck part (1) with an opening (1A), a wall part (2) and a bottom part (3),
**characterised in that** there are moulded onto the underside of the bottom part (3) one end of a first section (4A) and one end of a second section (4B) of an annular suspension device for suspending the receptacle, the free ends of the first and second section being able to be connected to one another by deformation of the sections to form the annular suspension device.

2. The parison according to claim 1, **characterised in that** the first and second section (4A, 4B) are constituted as flexible strips.

3. The parison according to claim 2, **characterised in that** the flexible strips (4A, 4B) are constituted as flat strips with a rectangular cross-section.

4. The parison according to any one of claims 1 to 3, **characterised in that** the ends of the first and second section (4A, 4B) moulded onto the underside of the bottom part (3) form a common base piece (4C), which is moulded onto the underside of the bottom part.

5. The parison according to claim 4, **characterised in that** the base piece (4C) is constituted as a flat body, which is moulded onto the underside of the bottom part (3).

6. The parison according to any one of claims 1 to 5, **characterised in that** the free ends of the first and second section (4A, 4B) are constituted as ends welded together, the end faces (4F, 4G) whereof are disposed lying opposite one another.

7. The parison according to claim 6, **characterised in that** the ends of the first and second section (4A, 4B) to be welded together each comprise an extension piece (4H, 41), the extension pieces of the end pieces being disposed with mutually facing contact faces (4J, 4K).

8. The parison according to any one of claims 1 to 5, **characterised in that** the first and second section (5A, 5B; 6A, 6B) are constituted as an annular body, wherein the sections overlap one another over a part of the periphery, so that the mutually facing faces of the sections form contact faces (5F, 5G; 6F, 6G) to be welded together.

9. The parison according to any one of claims 1 to 5, **characterised in that** the free ends of the first and second section (7A, 7B; 8A, 8B; 9A, 9B) are constituted as end pieces to be connected to one another in a keyed manner.

10. The parison according to claim 9, **characterised in that** the free ends of the first and second section (7A, 7B; 8A, 8B) are constituted as end pieces to be connected to one another in a keyed manner, the sections extending parallel to one another.

11. The parison according to claim 9, **characterised in that** the free ends of the first and second section (9A, 9B) are constituted as end pieces to be connected to one another in a keyed manner, the sections being is formed in an annular shape.

12. The parison according to claim 10 or 11, **characterised in that** the end piece of the first section (7A; 8A; 9A) and the end piece of the second section (7B; 8B; 9B) are constituted as a snap-in or catch connection.

13. The parison according to claim 12, **characterised in that** the end piece of the first section (8A) comprises a hook-shaped or claw-shaped body (8H) and the end piece (8B) of the second section (8A) comprises an undercut cutout (81), wherein the hook- or claw-shaped body of the first section can be inserted in the undercut cutout of the second section for the connection of the sections to form the annular suspension device.

14. Production of a receptacle used to hold medical fluids, in particular to hold infusion solutions or solutions for enteral nutrition, with the following process steps:
preparation of a parison in accordance with any one of claims 1 to 13,
stretching and blow-moulding of the parison to form a receptacle,
connection of the first and second section to form an annular suspension device.

15. The method according to claim 14, **characterised in that** the free ends of the first and second section are welded together.

16. The method according to claim 14, **characterised in that** the free ends of the first and second section are connected to one another in a keyed manner.

17. The method according to any one of claims 14 to 16, **characterised in that** the parison is produced in the injection moulding process.

18. A receptacle used to hold medical fluids, in particular to hold infusion solutions or solutions for enteral nutrition, **characterised in that** the receptacle is produced according to the method according to any one of claims 14 to 17.

## Revendications

1. Ébauche pour la fabrication d'un conteneur destiné à recevoir des liquides pour des applica-tions médicales, en particulier des solutions de perfusion ou solutions nutritives entérales, ladite ébauche comportant une partie de col (1) avec une ouverture (IA), une partie de paroi (2) et une partie de fond (3), **caractérisé en ce que**, sur la face inférieure de la partie de fond (3), sont formées la première extrémité d'une première pièce partielle (4A) et la première extrémité d'une deuxième pièce partielle (4B) d'un accro-choir annulaire pour la suspension du conteneur, les extrémités libres de la première et de la deuxième pièce partielle pouvant être raccordées l'une à l'autre en déformant les pièces par-tielles pour former l'accrochoir annulaire.

2. Ébauche selon la revendication 1, **caractérisée en ce que** la première et la deuxième pièce par-tielle (4a, 4B) sont réalisées comme lames flexibles.

3. Ébauche selon la revendication 2, **caractérisée en ce que** les lames flexibles (4a, 4B) sont réali-sées comme lames plates de section rectangulaire.

4. Ébauche selon l'une des revendications 1 à 3, **caractérisée en ce que** les extrémités de la première et de la deuxième pièce partielle (4a, 4B) formées sur la face inférieure de la partie de fond (3) constituent une pièce de base (4C) commune formée contre la face inférieure de la partie de fond.

5. Ébauche selon la revendication 4, **caractérisée en ce que** la pièce de base (4C) est réalisée comme un corps plat, formé contre la face inférieure de la partie de fond (3).

6. Ébauche selon l'une des revendications 1 à 5, **caractérisée en ce que** les extrémités libres de la première et de la deuxième pièce partielle (4a, 4B) sont réalisées comme extrémités à souder ensemble, dont les surfaces frontales (4F, 4G) sont opposées l'une à l'autre.

7. Ébauche selon la revendication 6, **caractérisée en ce que** les extrémités à souder ensemble de la première et de la deuxième pièce partielle (4A, 4B) comportent chacune une pièce d'épaulement (4H, 41), les pièces d'épaulement des pièces d'extrémité étant disposées avec des surfaces d'appui (4J, 4K) opposées l'une à l'autre.

8. Ébauche selon l'une des revendications 1 à 5, **caractérisée en ce que** la première et la deuxième pièce partielle (5A, 5B ; 6A, 6B) sont réalisées comme un corps annulaire, les pièces partielles se chevauchant sur une partie de la circonfé-rence, de telle façon que les surfaces opposées des pièces partielles forment des surfaces d'appui (5F, 5G ; 6F, 6G) à souder entre elles.

9. Ébauche selon l'une des revendications 1 à 5, **caractérisée en ce que** les extrémités libres de la première et de la deuxième pièce partielle (7A, 7B ; 8A, 8B ; 9A, 9B) sont réalisées comme pièces d'extrémité à raccorder par accouplement mécanique l'une à l'autre.

10. Ébauche selon la revendication 9, caractéri-sée en ce que les extrémités libres de la première et de la deuxième pièce partielle (7A, 7B ; 8A, 8B) sont réalisées comme pièces d'extré-mité à raccorder par accouplement mécanique l'une à l'autre, lesdites pièces partielles s'étendant parallèlement l'une à l'autre.

11. Ébauche selon la revendication 9, caractéri-sée en ce que les extrémités libres de la première et de la deuxième pièce partielle (9A, 9B) sont réalisées comme pièces d'extrémité à raccorder par accouplement mécanique l'une à l'autre, lesdites pièces partielles étant de forme annulaire.

12. Ébauche selon la revendication 10 ou la revendication 11, **caractérisée en ce que** la pièce d'extrémité de la première pièce partielle (7A ; 8A ; 9A) et la pièce d'extrémité de la deuxième pièce partielle (7B ; 8B ; 9B) sont réalisées comme une connexion à encliquetage ou à enclenchement.

13. Ébauche selon la revendication 12, caractéri-sée en ce que la pièce d'extrémité de la première pièce partielle (8A) présente un corps (8H) en forme de crochet ou de griffe, et la pièce d'extrémité (8B) de la deuxième pièce partielle (8A) un évidement (8I) à contre-dépouille, le corps en forme de crochet ou de griffe de la première pièce partielle pouvant être mis en place dans l'évidement à contre-dépouille de la deuxième pièce partielle pour raccorder les pièces partielles à l'accrochoir annulaire.

14. Fabrication d'un conteneur destiné à recevoir des liquides pour des applications médicales, en particulier des solutions de perfusion ou solu-tions nutritives entérales, au moyen des étapes de procédé suivantes :
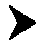 préparation d'une ébauche selon l'une des revendications 1 à 13,
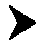 étirage et soufflage de l'ébauche pour former un conteneur,
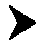 raccordement de la première et de la deuxième pièce partielle pour former un accrochoir annu-laire.

15. Procédé selon la revendication 14, caractéri-sé en ce que les extrémités libres de la première et de la deuxième pièce partielle sont soudées entre elles.

16. Procédé selon la revendication 14, caractéri-sé en ce que les extrémités libres de la première et de la deuxième pièce partielle sont raccordées par accouplement mécanique l'une à l'autre.

17. Procédé selon l'une des revendications 14 à 16, **caractérisé en ce que** l'ébauche est fabriquée par procédé de moulage par injection.

18. Conteneur destiné à recevoir des liquides pour des applications médicales, en particulier des solutions de perfusion ou solutions nutri-tives entérales, **caractérisé en ce que** ledit conteneur est fabriqué avec le procédé selon l'une des revendications 14 à 17.
